(19) Europäisches Patentamt
European Patent Office
Office européen des brevets

(11) EP 3 781 657 B1

(12) FASCICULE DE BREVET EUROPEEN

(45) Date de publication et mention de la délivrance du brevet:
**16.10.2024 Bulletin 2024/42**

(21) Numéro de dépôt: **19724559.0**

(22) Date de dépôt: **19.04.2019**

(51) Classification Internationale des Brevets (IPC):
***C11C 3/00*** *(2006.01)* ***C11C 3/10*** *(2006.01)*

(52) Classification Coopérative des Brevets (CPC):
**C11C 3/10; C11C 3/003**

(86) Numéro de dépôt international:
**PCT/FR2019/050937**

(87) Numéro de publication internationale:
**WO 2019/202276 (24.10.2019 Gazette 2019/43)**

(54) **UTILISATION D'ACIDE HYPOPHOSPHOREUX POUR L'ESTÉRIFICATION DES ACIDES GRAS LIBRES**

VERWENDUNG VON HYPOPHOSPHORIGER SÄURE ZUR VERESTERUNG FREIER FETTSÄUREN

USE OF HYPOPHOSPHOROUS ACID FOR THE ESTERIFICATION OF FREE FATTY ACIDS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **20.04.2018 FR 1853514**

(43) Date de publication de la demande:
**24.02.2021 Bulletin 2021/08**

(73) Titulaire: **ARKEMA FRANCE**
**92700 Colombes (FR)**

(72) Inventeur: **LAFFITTE, Jean-Alex**
**64200 BIARRITZ (FR)**

(74) Mandataire: **Casalonga**
**Casalonga & Partners**
**Bayerstraße 71/73**
**80335 München (DE)**

(56) Documents cités:
**EP-A1- 0 194 165**
**EP-A1- 0 194 165**
**WO-A1-95/01331**
**FR-A1- 2 929 621**
**US-A- 3 071 604**
**US-A- 3 887 488**
**US-A1- 2007 149 803**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 781 657 B1

## Description

La présente invention concerne les réactions d'estérification d'acides gras libres présents dans divers types d'huiles ou de graisses, qu'elles soient d'origine végétale, animale ou minérale.

La présente invention a également pour objet un procédé d'estérification des acides gras libres présents dans les huiles, notamment avant transformation desdites huiles en biocarburant, en particulier bio-diesel.

L'augmentation du coût des matières premières fossiles et l'évolution des normes environnementales rendent aujourd'hui nécessaire la recherche de nouvelles sources de matières premières. Parmi ces nouvelles sources, la biomasse constitue une alternative de choix et les huiles végétales, voire animales, sont déjà utilisées actuellement pour la fabrication de substances utiles dans le domaine de la cosmétique, des lubrifiants ou encore en tant que matières premières pour la production de biocarburant, notamment de bio-diesel.

Le bio-diesel est constitué en majeure partie d'esters méthyliques d'acides gras. Il est généralement produit par transestérification d'huiles végétales ou animales avec du méthanol, en présence de catalyseurs alcalins et notamment le méthylate de sodium (NaOMe), le méthylate de potassium (KOMe), l'hydroxyde de sodium (NaOH), l'hydroxyde de potassium (KOH) ou l'hydroxyde de calcium ($Ca(OH)_2$).

La réaction de transestérification peut être schématisée comme suit :

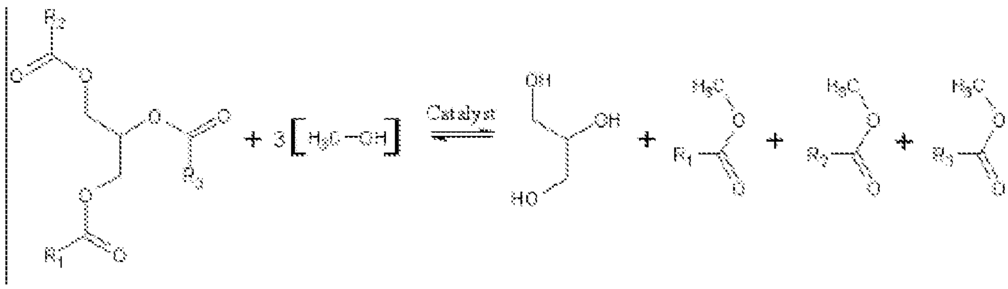


Huile méthanol glycérol biodiesel

Le biodiesel est ce mélange d'esters méthyliques d'acide gras.

Toutefois, la transestérification des huiles n'est pas sans poser de problèmes, notamment en raison des acides gras libres, présents dans lesdites huiles en quantité pouvant varier dans de très grandes proportions en fonction de l'origine même des huiles. Des quantités allant jusqu'à 40%, voire 70% d'acides gras libres ont été observées, notamment dans certaines huiles recyclées.

Or, à partir d'une teneur en acides gras libres dans une huile supérieure à 5%, voire supérieure à 10%, lesdits acides libres réagissent avec le catalyseur basique de transestérification pour former des savons (sels alcalins ou alcalino-terreux d'acide gras). Ces savons peuvent générer des émulsions et rendre difficile, voire impossible, la séparation du glycérol d'avec le bio-diesel.

Afin d'éviter la formation d'émulsions et de savons, et optimiser la séparation du glycérol dans le produit final, la teneur en acides gras libres avant transestérification devrait être aussi faible que possible, typiquement inférieure à 0,5% en poids, avantageusement inférieure à 0,1% en poids. Cette teneur devrait de préférence encore être de quelques ppm, idéalement la teneur en acides gras libres dans l'huile avant transestérification devrait être nulle.

Aujourd'hui, les acides gras libres sont typiquement estérifiés avec du méthanol, en présence d'un catalyseur acide. La littérature scientifique et la littérature brevets proposent une très grande variété de catalyseurs acides avec de très grandes disparités de résultats concernant le prétraitement des huiles afin de réduire, voire de rendre nul le taux d'acides gras libres avant la réaction de transestérification conduisant au biodiesel.

Les catalyseurs acides les plus communément utilisés aujourd'hui sont l'acide sulfurique ($H_2SO_4$) et l'acide paratoluènesulfonique (PTSA) et de nombreux articles scientifiques et publications de demandes de brevets divulguent des études d'estérifications des acides gras libres présents dans les huiles ou graisses végétales ou animales.

L'utilisation de l'acide sulfurique pose néanmoins d'importants problèmes de corrosion et d'effluents à traiter. Quant à l'acide paratoluènesulfonique, celui-ci contient par nature un cycle aromatique, et peut en outre contenir des impuretés aromatiques, ce qui le rend incompatible avec les normes environnementales relatives aux carburants, qui doivent précisément contenir une quantité aussi faible que possible, voire nulle, de composés aromatiques.

L'acide méthane sulfonique (AMS, ou MSA pour methane sulphonic acid en langue anglaise, ou encore sa forme anhydre AMSA pour Acide Méthane Sulfonique Anhydre) est également souvent proposé comme catalyseur d'estérification des acides gras libres. Il est connu de la demande FR 2 929 621 un procédé d'estérification d'acides gras libres utilisant l'AMS comme catalyseur acide dans des conditions opératoires facilement transposables à l'échelon industriel. Ce document divulgue également la préparation de bio-carburants efficaces et rentables.

Or, malgré les avantages, que présente ce procédé, il s'avère que l'estérification des acides gras libres avec du méthanol en présence d'AMS comme catalyseur génère une corrosion vis-à-vis de l'inox à 70°C.

Dans le cas d'une réaction de glycérolyse, qui consiste en l'estérification des acides gras libres avec du glycérol, conduite à 120-130°C, la vitesse de corrosion peut atteindre jusqu'à environ 600 μm/an à 130°C avec de l'AMS.

Il a été constaté, que la présence du catalyseur acide pour cette réaction d'estérification provoque une corrosion de l'inox, matériau des réacteurs utilisés. Or, si la réaction est menée en absence d'acide, afin de limiter la corrosion, la réaction d'estérification doit être conduite à 220°C, ce qui conduit à une consommation d'énergie beaucoup trop importante.

L'art antérieur précité montre clairement qu'il n'existe pas aujourd'hui de procédé permettant l'estérification des acides gras contenus dans le huiles ou graisses végétales ou animales, avec de bons rendements, et qui ne provoque pas de corrosion vis-à-vis des contenants en inox.

Par ailleurs, il est connu du document US 3,071,604 d'estérifier des acides gras en présence d'acide hypophosphoreux pour un but très différent de celui recherché, à savoir l'amélioration et la stabilité de la couleur des esters obtenus.

Dans un contexte encore différent, il est connu du document US 3,887,488 d'incorporer un dérivé de phosphore pour inhiber la corrosion d'acier inoxydable au sein de solution aqueuse d'acide sulfurique. WO95/01331 A1 décrit un procédé pour préparer un hydroxyalkylsulfonate d'ammonium en faisant réagir du bisulfite d'ammonium et un oxyde d'alkylène, où le pH est maintenu à une valeur relativement basse pendant la majeure partie de la réaction du bisulfite d'ammonium et de l'oxyde d'alkylène, afin de minimiser la quantité d'impuretés dans l'hydroxyalkylsulfonate d'ammonium.

Ainsi, un premier objectif de la présente invention consiste à fournir un inhibiteur de corrosion utilisable pour les procédés d'estérification d'acides gras libres.

Un autre objectif est de fournir un procédé d'estérification, qui mette en oeuvre un catalyseur acide n'engendrant que peu ou pas de corrosion des installations d'estérification et susceptible de conduire à la production de bio-carburant à faibles teneurs en composés aromatiques et en composés soufrés.

D'autres objectifs encore apparaîtront à la lumière de l'exposé de l'invention qui suit.

Il a en effet été découvert que l'utilisation d'acide hypophosphoreux pour les réactions d'estérification d'acides gras libres présents dans divers types d'huiles ou de graisses, qu'elles soient d'origine végétale, animale ou minérale présente l'avantage de limiter considérablement la corrosion des équipements en inox.

Il est précisé que les expressions "compris entre ... et ..." et « allant de ... à ... » utilisée dans le texte de la présente description et des revendications doit s'entendre comme incluant chacune des bornes mentionnées.

*Utilisation*

Ainsi, selon un premier aspect, la présente invention concerne l'utilisation d'acide hypophosphoreux en tant qu'inhibiteur de corrosion pour les réactions d'estérification d'acides gras libres présents dans divers types d'huiles ou de graisses, qu'elles soient d'origine végétale, animale ou minérale, en présence d'acide organo-sulfonique.

L'acide hypophosphoreux est de formule $H_3PO_2$.

L'acide hypophosphoreux utilisé peut être sous forme anhydre, c'est-à-dire à une teneur de 100% d'acide hypophosphoreux ou bien en solution aqueuse à 50%.

Dans la présente invention, on entend par acide organo-sulfonique les acides organo-sulfoniques de formule R-SO3H, dans laquelle R représente une chaîne hydrocarbonée saturée, linéaire ou ramifiée, comportant de 1 à 4 atomes de carbone.

La chaîne hydrocarbonée saturée, linéaire ou ramifiée, comportant de 1 à 4 atomes de carbone peut être substituée, en totalité ou en partie, par un ou plusieurs atomes d'halogène choisis parmi fluor, chlore et brome, et en particulier, la chaîne hydrocarbonée pouvant être perhalogénée, plus particulièrement perfluorée.

Ainsi, acides organo-sulfoniques compris dans le cadre de la présente invention sont de préférence choisis parmi l'acide méthane-sulfonique, l'acide éthane-sulfonique, l'acide n-propane-sulfonique, l'acide iso-propane-sulfonique, l'acide n-butane-sulfonique, l'acide iso-butane-sulfonique, l'acide sec-butane-sulfonique, l'acide tert-butane-sulfonique, l'acide trifluorométhanesulfonique, l'acide para-toluènesulfonique, l'acide benzènesulfonique et les mélanges de deux ou plusieurs d'entre eux, en toutes proportions.

Selon un mode de réalisation tout particulièrement préféré, l'acide organo-sulfonique utilisé dans le cadre de la présente invention est choisi parmi l'acide méthane-sulfonique, l'acide éthane-sulfonique, l'acide trifluorométhanesulfonique et l'acide para-toluènesulfonique et leur mélange. De manière tout à fait préférée, l'acide utilisé est l'acide méthane-sulfonique ou bien l'acide para-toluènesulfonique, et plus particulièrement l'acide méthane-sulfonique.

L'acide méthane sulfonique utilisé peut être sous forme anhydre, c'est-à-dire 100% d'acide méthane sulfonique, également dénommé AMSA pour anhydrous methane sulphonic acid en langue anglaise, ou encore sous forme de solution aqueuse, par exemple sous forme de solution à 70% en poids dans l'eau, commercialisée par la société Arkema.

Les réactifs de la réaction d'estérification, à savoir la substance grasse, l'alcool et l'acide gras libre sont ceux

définis ci-après pour le procédé selon l'invention.

*Composition*

L'invention porte également sur une composition comprenant

- de l'acide hypophosphoreux et
- de l'acide organo-sulfonique,

lesdits acides se trouvant dans un rapport massique acide hypophosphoreux sur acide organo-sulfonique allant de 0,01 à 0,4, de préférence de 0,01 à 0,3, et plus particulièrement de 0,01 à 0,25, et

- éventuellement un diluant aqueux, organique, ou hydro-organique, de préférence de l'eau, et
- éventuellement un ou plusieurs additifs.

Les additifs et les diluants sont avantageusement inertes vis-à-vis de la réaction d'estérification et de préférence également vis-à-vis de la réaction subséquente de transestérification.
Le diluant utilisable dans la composition est avantageusement choisi parmi les huiles, les éthers, les cétones, l'eau, du bio-diesel. Des mélanges de deux ou plusieurs des diluants ci-dessus peuvent également être utilisés.
Le diluant ne peut pas être un alcool, un acide, ni un ester. En effet, ces composés ne seraient pas inertes vis-à-vis de la réaction d'estérification.
Les éventuels additifs présents dans la composition peuvent être également de tout type, et par exemple choisis parmi les stabilisants, les modificateurs de rhéologie, les modificateurs de viscosité, les retardateurs de flamme, les tensio-actifs, les colorants, les conservateurs, les inhibiteurs de corrosion, les agents odorisants, et autres, ainsi que les mélanges de deux ou plusieurs d'entre eux.
Une composition selon l'invention particulièrement préférée comprend :

- de 1% à 20% en poids d'acide hypophosphoreux par rapport au poids total d'acide hypophosphoreux et d'acide sulfonique et
- de 80% à 99%, en poids d'acide organo-sulfonique et ses dérivés par rapport au poids total d'acide hypophosphoreux et d'acide organo-sulfonique.

Selon un mode de réalisation particulier de l'invention, la composition comprend

- de 5 à 20% en poids, de préférence de 8 à 15% en poids par rapport au poids total de la composition d'acide hypophosphoreux,
- de 30 à 70% en poids, de préférence de 40 à 60% en poids par rapport au poids total de la composition d'acide méthane sulfonique,

lesdits acides se trouvant dans un rapport massique acide hypophosphoreux sur acide organo-sulfonique allant de 0,01 à 0,4, de préférence de 0,01 à 0,3, et plus particulièrement de 0,01 à 0,25 et

- de 20 à 40% en poids par rapport au poids total de la composition d'eau.

*Procédé*

L'invention a également pour objet un procédé d'estérification d'acides gras libres compris dans une substance grasse, comprenant les étapes successives suivantes :

(a) fourniture d'une substance grasse comprenant de 1% à 100% en poids d'au moins un acide gras ;
(b) ajout d'au moins un alcool ;
(c) ajout d'une composition telle que définie ci-dessus, c'est-à-dire une composition comprenant

- de l'acide hypophosphoreux et
- de l'acide organo-sulfonique,

lesdits acides se trouvant dans un rapport massique acide hypophosphoreux sur acide organo-sulfonique allant de 0,01 à 0,4, de préférence de 0,01 à 0,3, et plus particulièrement de 0,01 à 0,25, et

- éventuellement un diluant aqueux, organique, ou hydro-organique, de préférence de l'eau, et
- éventuellement un ou plusieurs additifs.

(d) conduite de la réaction d'estérification à une température comprise entre la température ambiante et 150°C, de préférence entre 50°C et 140°C, plus préférentiellement entre 60°C et 130°C, sous pression atmosphérique ou sous un vide allant jusqu'à 1 kPa, de préférence entre 1 kPa et 100 kPa, de préférence entre 1 kPa et 50 kPa, de préférence de 5 kPa et 25 kPa.

Le procédé selon l'invention peut comprendre une étape supplémentaire, après l'étape (d) : étape (e) de récupération de la phase organique comprenant des esters d'acide gras.

Le procédé selon l'invention peut comprendre une étape (e) de récupération de la phase organique comprenant moins de 10%, de préférence moins de 6%, de préférence encore moins de 5%, mieux encore moins de 2% en poids d'acides gras libres.

Selon un mode de réalisation selon l'invention, on procède à l'élimination de l'eau contenue dans l'alcool, qui peut nuire à la réaction subséquente de transestérification en milieu basique.

Ainsi, l'étape (e) optionnelle peut se dérouler après une étape de séparation de l'alcool d'estérification et de l'eau.

La séparation de l'alcool d'estérification et de l'eau est effectuée selon des techniques classiques connues de l'homme du métier, telles que la distillation, décantation, évaporation partielle ou totale et/ou ajout éventuel de glycérol additivé d'hydroxyde de sodium, puis séparation des phases par décantation.

La substance grasse utilisée comme matière première à l'étape a) peut être de tout type connu en soi. Ce peut être tout type d'huile ou graisse d'origine végétale ou animale, ainsi que les mélanges desdites huiles et graisses, ou encore un acide gras libre ou un mélange d'acides gras libres, éventuellement en mélange avec une ou plusieurs huiles ou graisses d'origine végétale ou animale.

Parmi les huiles ou graisses végétales, on peut citer à titre d'exemples non limitatifs, les huiles de tournesol, de noix, de maïs, de soja, d'arachides, de colza, de lin, de chanvre, de carthame, de chardon marie, de pourpier, de framboise, de cassis, de noisette, d'amande, de germe de blé, de bourrache, d'onagre (primerose), d'oeillette, de noyaux, pépins et graines (par exemple noyaux d'abricots, amandes, pêches, cerises, ou prunes, pépins de raisin, pépins de courge ou de citrouille, graines de coton), d'argan, de palme, de coprah, de sésame, d'olive, de ricin, de noix de coco, de noix du Brésil, de jatropha, de babassu, de karanja, de neem, de mahua, de saijar, de jojoba, de tung, de tall ou tall oil, d'algues, ainsi que les huiles de friture usagées (Used Frying Oils, UFO ou encore Used cooking oil, UCO en langue anglaise), et autres.

Parmi les huiles ou graisses animales, on peut citer à titre d'exemples non limitatifs les huiles et graisses de poisson, telles que huile de foie de morue, huile de baleine, de cachalot, de dauphin, de phoque, de sardine, de hareng, de squales, les huiles et graisses de bovins, porcins, caprins, équidés, et volailles, telles que suif, saindoux, graisse de lait, lard, graisses de poulet, de boeuf, de porc, de cheval, et autres.

Les substances grasses décrites ci-dessus comprennent généralement un ou plusieurs acides gras dits libres, par comparaison avec les acides gras sous forme d'esters avec le glycérol. Parmi les acides gras rencontrés dans les huiles ou graisses mises en oeuvre dans le procédé de l'invention, on peut citer, à titre d'exemples non limitatifs, les acides gras libres sont choisis parmi les acides carboxyliques possédant de 3 à 32 atomes de carbone saturés ou insaturés, linéaires ou ramifiés, éventuellement comportant un ou plusieurs cycles. De préférence, les acides gras libres sont choisis parmi les acides carboxyliques possédant de 6 à 32 atomes de carbone, avantageusement de 8 à 32 atomes de carbone, en particulier de 10 à 32 atomes de carbone, de manière très préférée de 12 à 32 atomes de carbone.

De préférence, les acides carboxyliques choisi parmi l'acide propionique (3), l'acide butyrique (4), l'acide caproïque (6), l'acide caprylique (8), l'acide pélargonique (9), l'acide caprique (10), l'acide undécanoïque (11), l'acide laurique (12), l'acide myristique (14), l'acide myristoléique (14:1), l'acide palmitique (16), l'acide palmitoléique (16:1), l'acide margarique (17), l'acide stéarique (18), l'acide oléique (18:1), l'acide ricinoléique (18:1), l'acide élaïdique (18:1), l'acide pétrosélinique (18:1), l'acide vaccénique (18:1), l'acide linoléique (18:2), les acides linoléniques (18:3) et leurs isomères, l'acide licanique (18:3), l'acide arachidique (20), l'acide gadoléique (20:1), l'acide arachidonique (20:4), l'acide béhénique (22), l'acide érucique (C22:1), ainsi que les mélanges de deux ou plusieurs d'entre eux, en toutes proportions.

Comme indiqué précédemment, la substance grasse mise en oeuvre dans le procédé de l'invention comprend de 0,1% à 100%, de préférence de 1% à 99%, de préférence encore de 3% à 95%, de manière tout à fait préférée, de 5% à 90% en poids d'au moins un acide gras libre. La substance grasse peut par exemple être constituée uniquement par un acide gras libre, ou un mélange d'acides gras libres, ou encore comprendre un mélange d'au moins un acide gras avec une ou plusieurs huiles et/ou graisses végétales ou animales.

À la substance grasse est ajoutée une quantité efficace d'au moins un alcool, pour l'estérification des acides gras.

Les alcools pouvant être utilisés peuvent être choisis parmi les monoalcools, les diols, et triols alkyliques, linéaires, ramifiés ou cycliques, de préférence linéaires ou ramifiés, de préférence encore linéaires, comprenant de 1 à 6 atomes de carbone, de préférence de 1 à 4 atomes de carbone, et les mélanges de deux ou plusieurs d'entre eux,

en toutes proportions.

Les alcools pouvant être utilisés peuvent également être choisis parmi les monoalcools et les polyols, de préférence parmi les monoalcools et les polyols alkyliques par exemple parmi les monoalcools, les diols, les triols et les tétraols alkyliques, linéaires, ramifiés ou cycliques, de préférence linéaires ou ramifiés, de préférence encore linéaires, comprenant de 1 à 6 atomes de carbone, de préférence de 1 à 5 atomes de carbone, et les mélanges de deux ou plusieurs d'entre eux, en toutes proportions. Par exemple, l'alcool peut être choisi parmi le méthanol, l'éthanol, le propanol, l'éthylèneglycol, le glycérol, le pentaérythritol et leurs mélanges.

De préférence, l'alcool est choisi parmi le méthanol, l'éthanol, propanol, éthylèneglycol, et le glycérol et leurs mélanges, de préférence, l'alcool est le méthanol ou le glycérol.

L'alcool particulièrement préféré dans le procédé de l'invention est le méthanol.

Des mélanges de deux ou plusieurs des alcools précités sont également envisageables.

Avantageusement, le méthanol et le glycérol sont les alcools mis en oeuvre dans le procédé selon l'invention.

Bien qu'il soit possible d'introduire dans la substance grasse de départ une quantité molaire équivalente d'alcool par rapport au nombre de moles d'acides gras à estérifier, on préfère ajouter un excès molaire d'alcool. Ainsi, le nombre de moles d'alcool pourra être compris entre 1,1 et 50 fois, de préférence entre 3 et 47 fois, de préférence encore entre 5 et 25 fois, par exemple 10 fois, le nombre de moles d'acides gras à estérifier. Une quantité supérieure à 50 ne serait pas rentable sur le plan économique et des volumes à traiter. Une quantité stoechiométrique d'alcool pourrait conduire à une estérification incomplète des acides gras libres.

De préférence, le rapport molaire entre le mélange d'acides selon l'invention : acide hypophosphoreux et acide organo-sulfonique sur les acides gras libres est compris entre 0.001 et 10, de préférence entre 0.05 et 1.

La réaction d'estérification est conduite, de préférence sous agitation à une température comprise entre la température ambiante et 150°C, de préférence entre 50°C et 140°C, plus préférentiellement entre 60°C et 130°C.

La réaction est conduite sous pression atmosphérique ou sous un vide allant jusqu'à 1 kPa, de préférence entre 1 kPa et 100 kPa, de préférence entre 1 kPa et 50 kPa, de préférence de 5 kPa et 25 kPa.

La réaction d'estérification est généralement conduite pendant une durée variant, selon les conditions opératoires entre quelques dizaines de minutes et plusieurs heures. Habituellement, la réaction est conduite jusqu'à ce que la quantité d'acides gras libres au sein de la substance grasse chargée soit inférieure à 10% en poids, de préférence inférieure à 6% en poids, plus particulièrement inférieure à 5% en poids, de manière encore plus avantageuse inférieure à 2% en poids.

En règle générale, selon le procédé de la présente invention, une durée réactionnelle comprise entre environ 20 minutes et 5 heures est suffisante pour obtenir un taux de conversion voisin de 100%.

Après la réaction d'estérification, l'excès éventuel d'alcool, utilisé pour l'estérification des acides gras libres (tel que méthanol ou le glycérol) et l'eau, peuvent être éliminés du milieu réactionnel selon des méthodes classiques de séparation, telles que distillation, décantation, évaporation partielle ou totale, deux ou plusieurs de ces techniques pouvant être combinées entre elles.

Selon une alternative, l'alcool en excès peut être recyclé dans l'opération d'estérification ou dans l'opération de transestérification, après avoir été débarrassé de l'eau qu'il contient, selon des techniques classiques d'élimination d'eau dans les alcools. En outre, le catalyseur peut éventuellement être neutralisé par ajout d'une base telle que l'hydroxyde de potassium, puis filtré après évaporation de la phase méthanol-eau.

En fin de procédé, le milieu réactionnel issu de l'étape d) peut comprendre une quantité résiduelle d'acides gras libres inférieure à 10% en poids, de préférence inférieure à 6% en poids, plus particulièrement inférieure à 5% en poids, de manière encore plus avantageuse inférieure à 2% en poids, idéalement inférieure à 0,5% en poids, de préférence inférieure à 0,1 % en poids.

Le milieu réactionnel peut être soumis à diverses opérations de neutralisation, lavage, filtration, afin d'obtenir un mélange d'esters d'acides gras.

Cette opération a pour avantage d'être adapté à une éventuelle réaction subséquente de transestérification.

Selon une autre variante du procédé selon l'invention, le milieu réactionnel issu de l'étape d) peut être échangé directement dans une autre réaction, tel que par exemple une réaction de transestérification. En d'autres termes, le milieu réactionnel issu de l'étape d) peut être utilisé tel quel, c'est-à-dire sans traitement spécifique. Un procédé monotope, sans séparation des produits de réaction peut être envisagé.

En raison du faible, voire très faible taux d'acides gras libres présents dans le milieu réactionnel, celui-ci peut être engagé dans un procédé de préparation de bio-diesel, c'est-à-dire une réaction de transestérification des triglycérides d'acides gras, avec un alcool, de préférence le méthanol. Cette réaction de transestérification est généralement effectuée en présence d'un catalyseur basique selon des techniques maintenant bien connues de l'homme du métier.

Il est en effet connu que toutes les huiles ou graisses, d'origine végétale ou animale, peuvent être utilisées pour la fabrication de bio-carburant, plus précisément de bio-diesel, comme indiqué plus haut.

Le bio-diesel présente de très nombreux avantages, et parmi ceux-ci, on peut citer, sa très faible teneur en produits soufrés, sa très faible teneur en produits aromatiques, sa biodégradabilité très rapide par rapport au carburants

d'origine fossile, son fort pouvoir lubrifiant qui permet de réduire l'usure prématurée des moteurs.

La préparation de bio-diesel par transestérification, en présence d'un catalyseur basique, est par exemple décrite dans les demandes de brevets WO 2006/043281 et US 2003/0167681. Ce procédé peut être appliqué aux huiles et graisses animales et/ou végétales, qui ont subi le procédé précédemment décrit d'estérification des acides gras libres.

L'invention a pour autre objet un procédé de préparation de bio-diesel, mettant en oeuvre la composition telle que définie ci-dessus, c'est-à-dire une composition telle que définie ci-dessus, c'est-à-dire une composition comprenant

- de l'acide hypophosphoreux et
- de l'acide organo-sulfonique,

lesdits acides se trouvant dans un rapport massique acide hypophosphoreux sur acide organo-sulfonique allant de 0,01 à 0,4, de préférence de 0,01 à 0,3, et plus particulièrement de 0,01 à 0,25, et

- éventuellement un diluant aqueux, organique, ou hydro-organique, de préférence de l'eau, et
- éventuellement un ou plusieurs additifs.

De préférence, le procédé de préparation de bio-diesel selon l'invention comporte les étapes de procédé telles que définies ci-dessus, c'est-à-dire les étapes successives suivantes :

(a) fourniture d'une substance grasse comprenant de 1% à 100% en poids d'au moins un acide gras ;
(b)ajout d'au moins un alcool ;
(c) ajout d'une composition telle que définie ci-dessus, c'est-à-dire une composition comprenant

- de l'acide hypophosphoreux et
- de l'acide organo-sulfonique,

lesdits acides se trouvant dans un rapport massique acide hypophosphoreux sur acide organo-sulfonique allant de 0,01 à 0,4, de préférence de 0,01 à 0,3, et plus particulièrement de 0,01 à 0,25, et

- éventuellement un diluant aqueux, organique, ou hydro-organique, de préférence de l'eau, et
- éventuellement un ou plusieurs additifs.

(d) conduite de la réaction d'estérification à une température comprise entre la température ambiante et 150°C, de préférence entre 50°C et 140°C, plus préférentiellement entre 60°C et 130°C, sous pression atmosphérique ou sous un vide allant jusqu'à 1 kPa, de préférence entre 1 kPa et 100 kPa, de préférence entre 1 kPa et 50 kPa, de préférence de 5 kPa et 25 kPa.

Plus précisément, le brut réactionnel obtenu à l'étape (e) du procédé d'estérification des acides gras libres décrit précédemment peut être engagé dans un procédé de préparation de bio-diesel comprenant les étapes suivantes :

(f) filtration éventuelle du produit de l'étape (e) ;
(g) ajout d'un catalyseur basique et d'au moins un alcool ;
(h) conduite de la réaction de transestérification à une température comprise entre 30°C et 300°C, à une pression comprise entre 10 kPa et 1000 kPa ;
(i) récupération, et éventuellement séchage et filtration, des esters d'acides gras transestérifiés.

Il peut être avantageux de purifier le brut réactionnel d'estérification des acides gras libres, afin d'éliminer les espèces pouvant perturber la réaction subséquente de transestérification. Ainsi il est possible de procéder à une filtration, selon des techniques classiques connues de l'homme du métier, mais aussi à une distillation, un séchage, et autres techniques connues de raffinage de bruts réactionnels.

Le catalyseur basique est connu en soi et peut être avantageusement choisi parmi les catalyseurs basiques couramment utilisés dans ce domaine, c'est-à-dire parmi hydroxyde de sodium, hydroxyde de potassium, méthylate de sodium, méthylate de potassium, carbonate de sodium, carbonate de potassium, carbonate de calcium, et autres. Le catalyseur basique peut être supporté, sur des résines, des zéolites, des oxydes métalliques (alumine, silice, zircone, etc.). Selon un autre aspect, la réaction de transestérification peut être conduite en l'absence de catalyseur basique.

L'alcool ou les mélanges d'alcools, qui peuvent être utilisés sont généralement choisis parmi les mono-alcools alkyliques, par exemple le méthanol, l'éthanol, le propanol et le butanol. La quantité d'alcool(s) introduite est avantageusement telle que le milieu réactionnel comprend un excès molaire d'alcool(s) par rapport aux nombre de moles

d'esters, afin de favoriser la réaction de transestérification.

Le brut réactionnel de transestérification contient majoritairement des esters d'acides gras (bio-diesel) et du glycérol qui est séparé selon des techniques classiques. Les esters d'acides gras peuvent enfin être éventuellement séchés, purifiés, filtrés, selon des techniques classiques avant de pouvoir être utilisés comme bio-carburants.

Les exemples suivants illustrent la présente invention.

Dans ces exemples, les parties et les pourcentages sont exprimés en poids, sauf indication contraire.

EXEMPLES

Les expériences suivantes ont été menées :

1. Estérifications comparatives notées réactions A à C

Un réacteur double enveloppe de 500 mL fermé par un chapeau comportant 6 sorties, est équipé d'une sonde thermométrique, d'une agitation mécanique, d'un septum qui pourra être remplacé en début de manipulation par une ampoule de coulée pour l'ajout du catalyseur, d'une tige en verre porte-coupons avec 3 coupons en inox 316L et d'un tube plongeur avec diffuseur. Sur le dernier col, il y a un réfrigérant droit de 15 cm refroidi à 10°C suivi d'un col de cygne avec une sortie d'air, puis d'une ampoule à décanter graduée (5mL, 10mL, 15mL). La sortie d'air au bout du réfrigérant est reliée à un piège à gaz puis à la pompe à vide.

Au préalable, les dimensions et la masses des 3 coupons en inox sont mesurées. L'inox est de l'inox 316L de densité 7,99 $g/cm^3$.

Le réacteur est préchauffé à 60°C. 226g d'huile à 93,5% d'acide gras libre, ci-après noté FFA, sont introduits rapidement dans le réacteur chaud (60°C). Une faible agitation (120rpm) est appliquée. 81g de glycérol sont ensuite ajoutés à l'huile dans le réacteur. Quand l'ajout du glycérol est fini, l'agitation est augmentée à 500 rpm. Le réacteur est mis sous azote avec dégazage du milieu grâce à un tube plongeur. La température est augmentée de 60 à 130°C sur environ 30 minutes. A partir de 120°C, l'inertage est laissé 15 minutes.

Le ou les acides sont alors ajoutés à l'aide d'une aiguille à travers le septum.

Les 3 réactions comparatives d'estérification notées A, B et C sont menées avec différents acides figurant dans le tableau ci-dessous :

| | Acides |
|---|---|
| A | 0,5% d'AMS seul, soit 1,11g d'AMS à 70%, soit 0,5% par rapport à l'huile |
| B | 0,5% de $H_2SO_4$ seul, soit 1.11 g d' $H_2SO_4$ à 96 %, soit 0,5% par rapport à l'huile |
| C | un mélange de 0,07% de $H_3PO_2$ et 0,5% de $H_2SO_4$, soit 0,16g d'acide hypophosphoreux à 50%, soit 0,07% par rapport à l'huile et 1.11g d' $H_2SO_4$ à 96 %, soit 0,5% par rapport à l'huile |

Le réacteur est alors mis sous vide de 1 kPa et le milieu réactionnel est agité pendant 6,5 heures à 130°C. En fin de réaction, le milieu est refroidi à 40°C, puis mis sous air et décanté dans une ampoule à décanter : la phase organique se trouve en haut et la phase aqueuse contenant le glycérol se trouve en bas. 263g de phase organique sont obtenus.

Les 3 coupons en inox 316L sont plongés dans de l'eau ultra pure puis lavés à l'éthanol, puis séchés à l'air et pesés.

2. Estérification selon l'invention notée réaction D

Le mode opératoire décrit ci-dessus est suivi. Suite à l'étape de mélange des réactifs, 1,11g d'AMS à 70%, soit 0,5% par rapport à l'huile, additivé de 0,16g d'acide hypophosphoreux à 50%, soit 0,07% par rapport à l'huile, soit un ratio massique $H_3PO_2$ exprimé en équivalent anhydre sur AMS 70% de 0.08/0.77= 0.1 est alors ajouté à l'aide d'une aiguille à travers le septum. Le réacteur est alors mis sous vide de 1 kPa et le milieu réactionnel est agité pendant 6,5 heures à 130°C. En fin de réaction, le milieu est refroidi à 40°C, puis mis sous air et décanté dans une ampoule à décanter : la phase organique se trouve en haut et la phase aqueuse contenant le glycérol se trouve en bas. 263g de phase organique sont obtenus.

Les 3 coupons en inox 316L sont plongés dans de l'eau ultra pure puis lavés à l'éthanol, puis séchés à l'air et pesés.

3. Résultats

Pour chaque procédé suivi, la perte de masse des coupons en inox présents dans le réacteur est calculée. Cette perte de masse permet de calculer la vitesse de corrosion en µm/an selon la formule suivante:

$$V = [\Delta p/(d \times S \times 10^{-4} \times t)] \times 365$$

avec V = vitesse de corrosion exprimée en µm/an
Δp = perte de masse du coupon testé exprimée en g,
d = densité du matériau du coupon testé exprimée en g/cm$^3$
S = surface du coupon testé exprimée en cm$^2$
t = durée de la manipulation exprimée en jour

Le tableau ci-dessous résume les résultats obtenus pour les réactions d'estérification comparatives et avec le mélange selon l'invention.

| | Acides | Vitesse de corrosion (µm/an) | Conversion (%) |
|---|---|---|---|
| A Comparative | AMS | 585 | > 95 |
| B Comparative | $H_2SO_4$ | 900 | > 95 |
| C Comparative | $H_3PO_2$ + $H_2SO_4$ | 515 | 87 |
| D Invention | $H_3PO_2$ + AMS | 35 | > 95 |

Les résultats montrent très clairement que la combinaison des deux acides tels que revendiqués, à savoir l'acide hypophosphoreux et l'acide méthane sulfonique conduit à une très forte diminution de la corrosion, tout en conduisant à un fort taux de conversion.

**Revendications**

1. Utilisation d'acide hypophosphoreux en tant qu'inhibiteur de corrosion pour les réactions d'estérification d'acides gras libres, en présence d'acide organo-sulfonique de formule R-SO3H, dans laquelle R représente une chaîne hydrocarbonée saturée, linéaire ou ramifiée, comportant de 1 à 4 atomes de carbone.

2. Utilisation selon la revendication 1, **caractérisée en ce que** l'acide organo-sulfonique est l'acide méthane sulfonique.

3. Composition comprenant

   - de l'acide hypophosphoreux et
   - de l'acide organo-sulfonique,

   lesdits acides se trouvant dans un rapport massique acide hypophosphoreux sur acide organo-sulfonique allant de 0,01 à 0,4, de préférence de 0,01 à 0,3, et plus particulièrement de 0,01 à 0,25, et

   - éventuellement un diluant aqueux, organique, ou hydro-organique, de préférence de l'eau, et
   - éventuellement un ou plusieurs additifs.

4. Composition selon la revendication 3, **caractérisée en ce qu'**elle comprend :

   - de 1% à 20% en poids d'acide hypophosphoreux par rapport au poids total d'acide hypophosphoreux et d'acide sulfonique et
   - de 80% à 99%, en poids d'acide organo-sulfonique par rapport au poids total d'acide hypophosphoreux et d'acide organo-sulfonique.

5. Procédé d'estérification d'acides gras, **caractérisé en ce qu'**il comprend les étapes successives suivantes :

(a) fourniture d'une substance grasse comprenant de 1% à 100% en poids d'au moins un acide gras ;
(b) ajout d'au moins un alcool ;
(c) ajout d'une composition telle que définie à la revendication 3 ou 4 ;
(d) conduite de la réaction d'estérification à une température comprise entre la température ambiante et 150°C, de préférence entre 50°C et 140°C, plus préférentiellement entre 60°C et 130°C, sous pression atmosphérique ou sous un vide allant jusqu'à 1 kPa, de préférence entre 1 kPa et 100 kPa, de préférence entre 1 kPa et 50 kPa, de préférence de 5 kPa et 25 kPa.

6. Procédé selon la revendication 5, **caractérisé en ce qu'**il comporte, après l'étape (d), l'étape suivante :
(e) récupération de la phase organique comprenant des esters d'acide gras.

7. Procédé selon la revendication 5 ou 6, **caractérisé en ce que** la substance grasse est une huile ou une graisse d'origine végétale ou animale, ou une huile de friture usagée, ou un mélanges des huiles ou graisses précitées, ou encore un acide gras libre ou un mélange d'acides gras libres, éventuellement en mélange avec une ou plusieurs huiles ou graisses d'origine végétale ou animale.

8. Procédé selon l'une quelconque des revendications 5 à 7, **caractérisé en ce que** la substance grasse est choisie parmi les huiles de tournesol, de noix, de maïs, de soja, d'arachides, de colza, de lin, de chanvre, de carthame, de chardon marie, de pourpier, de framboise, de cassis, de noisette, d'amande, de germe de blé, de bourrache, d'onagre (primerose), d'oeillette, de noyaux, pépins et graines, d'argan, de palme, de coprah, de sésame, d'olive, de ricin, de noix de coco, de noix du Brésil, de jatropha, de babassu, de karanja, de neem, de mahua, de saijar, de jojoba, de tung, de tall ou «tall oil », d'algues, les huiles de friture usagées, les huiles et graisses de poisson, les huiles et graisses de bovins, porcins, caprins, équidés, et volailles, ainsi que les mélanges de deux ou plusieurs d'entre elles.

9. Procédé selon l'une quelconque des revendications 5 à 8, **caractérisé en ce que** la substance grasse comprend de 0,1% à 100%, de préférence de 1% à 99%, de préférence encore de 3% à 95%, de manière tout à fait préférée, de 5% à 90% en poids d'au moins un acide gras libre.

10. Procédé selon l'une quelconque des revendications 5 à 9, **caractérisé en ce que** les acides gras libres sont choisis parmi les acides carboxyliques possédant de 3 à 32 atomes de carbone saturés ou insaturés, linéaires ou ramifiés, comportant éventuellement un ou plusieurs cycles, et notamment les acides carboxyliques choisi parmi l'acide propionique (3), l'acide butyrique (4), l'acide caproïque (6), l'acide caprylique (8), l'acide pélargonique (9), l'acide caprique (10), l'acide undécanoïque (11), l'acide laurique (12), l'acide myristique (14), l'acide myristoléique (14:1), l'acide palmitique (16), l'acide palmitoléique (16:1), l'acide margarique (17), l'acide stéarique (18), l'acide oléique (18:1), l'acide ricinoléique (18:1), l'acide élaïdique (18:1), l'acide pétrosélinique (18:1), l'acide vaccénique (18:1), l'acide linoléique (18:2), les acides linoléniques (18:3) et leurs isomères, l'acide licanique (18:3), l'acide arachidique (20), l'acide gadoléique (20:1), l'acide arachidonique (20:4), l'acide béhénique (22), l'acide érucique (C22:1), ainsi que les mélanges de deux ou plusieurs d'entre eux, en toutes proportions.

11. Procédé selon l'une quelconque des revendications 5 à 10, **caractérisé en ce que** l'alcool est choisi parmi les monoalcools, les diols, et triols alkyliques, linéaires, ramifiés ou cycliques, de préférence linéaires ou ramifiés, de préférence encore linéaires, comprenant de 1 à 6 atomes de carbone, de préférence de 1 à 4 atomes de carbone, et les mélanges de deux ou plusieurs d'entre eux, en toutes proportions.

12. Procédé selon la revendication 11, **caractérisé en ce que** l'alcool est choisi parmi le méthanol, l'éthanol, propanol, éthylèneglycol, et le glycérol et leurs mélanges, de préférence, l'alcool est le méthanol ou le glycérol.

13. Procédé de préparation de bio-diesel, **caractérisé en ce qu'**il met en oeuvre la composition telle que définie à la revendication 3 ou 4.

14. Procédé selon la revendication 13, **caractérisé en ce qu'**il comporte les étapes de procédé telles que définies à l'une quelconque des revendications 5 à 12.

## Patentansprüche

1. Verwendung von Hypophosphorsäure als Korrosionsinhibitor für Veresterungsreaktionen von freien Fettsäuren in Gegenwart von Organosulfonsäure der Formel R-SO3H, in der R eine gesättigte, lineare oder verzweigte Kohlenwasserstoffkette darstellt, die 1 bis 4 Kohlenstoffatome aufweist.

**2.** Verwendung nach Anspruch 1, **dadurch gekennzeichnet, dass** die Organosulfonsäure Methansulfonsäure ist.

**3.** Zusammensetzung, die Folgendes umfasst:

- Hypophosphorsäure und
- Organosulfonsäure,

wobei die Säuren in einem Massenverhältnis von Hypophosphorsäure zu Organosulfonsäure im Bereich von 0,01 bis 0,4, vorzugsweise von 0,01 bis 0,3 und im Besonderen von 0,01 bis 0,25 vorliegen, und

- gegebenenfalls ein wässriges, organisches oder hydroorganisches Verdünnungsmittel, vorzugsweise Wasser, und
- gegebenenfalls ein oder mehrere Hilfsstoffe.

**4.** Zusammensetzung nach Anspruch 3, **dadurch gekennzeichnet, dass** sie Folgendes umfasst:

- 1 bis 20 Gew.-% Hypophosphorsäure in Bezug auf das Gesamtgewicht von Hypophosphorsäure und Sulfonsäure und
- 80 bis 99 Gew.-% Organosulfonsäure in Bezug auf das Gesamtgewicht von Hypophosphorsäure und Organosulfonsäure.

**5.** Verfahren zur Veresterung von Fettsäuren, **dadurch gekennzeichnet, dass** es die folgenden aufeinanderfolgenden Schritte umfasst:

(a) Bereitstellen einer Fettsubstanz, die 1 Gew.-% bis 100 Gew.-% mindestens einer Fettsäure umfasst;
(b) Zugeben von mindestens einem Alkohol;
(c) Zugeben einer wie im Anspruch 3 oder 4 definierten Zusammensetzung;
(d) Durchführen der Veresterungsreaktion bei einer Temperatur zwischen der Raumtemperatur und 150 °C, vorzugsweise zwischen 50 °C und 140 °C, im Besonderen zwischen 60 °C und 130 °C, unter atmosphärischem Druck oder in einem Vakuum von bis zu 1 kPa, vorzugsweise zwischen 1 kPa und 100 kPa, vorzugsweise zwischen 1 kPa und 50 kPa, vorzugsweise 5 kPa und 25 kPa.

**6.** Verfahren nach Anspruch 5, **dadurch gekennzeichnet, dass** es nach Schritt (d) den folgenden Schritt umfasst: (e) Gewinnen der organischen Phase, die Fettsäureester umfasst.

**7.** Verfahren nach Anspruch 5 oder 6, **dadurch gekennzeichnet, dass** die Fettsubstanz ein Öl oder Fett pflanzlichen oder tierischen Ursprungs oder ein gebrauchtes Frittieröl oder eine Mischung aus den zuvor genannten Ölen oder Fetten oder auch ein freie Fettsäure oder eine Mischung aus freien Fettsäuren ist, gegebenenfalls eine Mischung mit einem oder mehreren Ölen oder Fetten pflanzlichen oder tierischen Ursprungs.

**8.** Verfahren nach einem der Ansprüche 5 bis 7, **dadurch gekennzeichnet, dass** die Fettsubstanz ausgewählt ist aus Sonnenblumenöl, Nussöl, Maisöl, Sojaöl, Erdnussöl, Rapsöl, Leinsamenöl, Hanföl, Färberdistelöl, Mariendistelöl, Portulaköl, Himbeeröl, Schwarze-Johannisbeer-Öl, Haselnussöl, Mandelöl, Weizenkeimöl, Borretschöl, Nachtkerzenöl (Primerose), Nelkenöl, Kernöl, Samen und Saatgut, Arganöl, Palmöl, Kopraöl, Sesamöl, Olivenöl, Rizinusöl, Kokosnussöl, Paranussöl, Jatrophaöl, Babassuöl, Karanjaöl, Niemöl, Mahuaöl, Saijaröl, Jojobaöl, Tungöl, Tallöl oder "Tall Oil", Algenöl, gebrauchten Frittierölen, Fischölen und -fetten, Ölen und Fetten von Rindern, Schweinen, Ziegen, Pferden und Geflügel sowie Mischungen aus zwei oder mehr davon.

**9.** Verfahren nach einem der Ansprüche 5 bis 8, **dadurch gekennzeichnet, dass** die Fettsubstanz 0,1 Gew.-% bis 100 Gew.-%, vorzugsweise 1 Gew.-% bis 99 Gew.-%, besonders bevorzugt 3 Gew.-% bis 95 Gew.-%, am meisten bevorzugt 5 Gew.-% bis 90 Gew.-% mindestens einer freien Fettsäure umfasst.

**10.** Verfahren nach einem der Ansprüche 5 bis 9, **dadurch gekennzeichnet, dass** die freien Fettsäuren ausgewählt sind aus Carbonsäuren mit 3 bis 32 gesättigten oder ungesättigten, linearen oder verzweigten Kohlenstoffatomen, die gegebenenfalls einen oder mehrere Ringe aufweisen, und insbesondere die Carbonsäuren ausgewählt sind aus Propionsäure (3), Buttersäure (4), Capronsäure (6), Caprylsäure (8), Pelargonsäure (9), Caprinsäure (10),

Undecansäure (11), Laurinsäure (12) , Myristinsäure (14), Myristoleinsäure (14:1), Palmitinsäure (16), Palmitoleinsäure (16:1), Margarinsäure (17), Stearinsäure (18), Ölsäure (18:1), Ricinolsäure (18:1), Elaidinsäure (18:1), Petroselinsäure (18:1), Vaccensäure (18:1), Linolsäure (18:2), Linolensäure (18:3) und deren Isomere, Licansäure (18:3), Arachinsäure (20), Gadoleinsäure (20:1), Arachidonsäure (20:4), Behensäure (22), Erucasäure (C22:1) sowie Mischungen aus zwei oder mehr davon in jedem beliebigen Verhältnis.

**11.** Verfahren nach einem der Ansprüche 5 bis 10, **dadurch gekennzeichnet, dass** der Alkohol ausgewählt ist aus linearen, verzweigten oder cyclischen Alkylmonoalkoholen, -diolen und -triolen, vorzugsweise linearen oder verzweigten, besonders bevorzugt linearen, umfassend 1 bis 6 Kohlenstoffatome, vorzugsweise 1 bis 4 Kohlenstoffatome, und Mischungen aus zwei oder mehr davon in jedem beliebigen Verhältnis.

**12.** Verfahren nach Anspruch 11, **dadurch gekennzeichnet, dass** der Alkohol ausgewählt ist aus Methanol, Ethanol, Propanol, Ethylenglykol und Glycerin und deren Mischungen, vorzugsweise ist der Alkohol Methanol oder Glycerin.

**13.** Verfahren zum Herstellen von Biodiesel, **dadurch gekennzeichnet, dass** dabei die wie Anspruch 3 oder 4 definierte Zusammensetzung verwendet wird.

**14.** Verfahren nach Anspruch 13, **dadurch gekennzeichnet, dass** es die wie in einem der Ansprüche 5 bis 12 definierten Verfahrensschritte aufweist.

## Claims

**1.** Use of hypophosphorous acid as a corrosion inhibitor for esterification reactions on free fatty acids, in the presence of organosulfonic acid of formula $R-SO_3H$, wherein R represents a saturated hydrocarbon chain, linear or branched, including 1 to 4 carbon atoms.

**2.** Use according to claim 1, **characterised in that** the organosulfonic acid is methanesulfonic acid.

**3.** Composition comprising

- hypophosphorous acid, and
- organosulfonic acid,

said acids being in a ratio by mass of hypophosphorous acid to organosulfonic acid ranging from 0.01 to 0.4, preferably from 0.01 to 0.3, and more particularly from 0.01 to 0.25, and

- optionally an aqueous diluent, organic, or hydroorganic, preferably water, and
- optionally one or more additives.

**4.** Composition according to claim 3, **characterised in that** it comprises:

- from 1% to 20% by weight hypophosphorous acid with respect to the total weight of hypophosphorous acid and sulfonic acid, and
- from 80% to 99% by weight organosulfonic acid with respect to the total weight of hypophosphorous acid and organosulfonic acid.

**5.** Method for the esterification of fatty acids, **characterised in that** it comprises the following successive steps:

(a) providing a fatty substance comprising from 1% to 100% by weight at least one fatty acid;
(b) adding at least one alcohol;
(c) adding a composition as defined in claim 4 or 5;
(d) conducting the esterification reaction at a temperature lying between ambient temperature and 150°C, preferably between 50°C and 140°C, more preferentially between 60°C and 130°C, at atmospheric pressure or under a vacuum ranging up to 1 kPa, preferably between 1 kPa and 100 kPa, preferably 1 kPa and 50 kPa, preferably from 5 kPa to 25 kPa.

**6.** Method according to claim 5, **characterised in that** it includes, after step (d), the following step:

(e) recovering the organic phase comprising fatty acid esters.

7. Method according to claim 5 or 6, **characterised in that** the fatty substance is an oil or a fat of vegetable or animal origin, or a used frying oil, or a mixture of the aforementioned oils or fats, or a free fatty acid or a mixture of free fatty acids, optionally a mixture with one or more oils or fats of vegetable or animal origin.

8. Method according to any one of claims 5 to 7, **characterised in that** the fatty substance is chosen from oils of sunflower, walnut, maize, soya, peanuts, colza, linseed, hemp, safflower, milk thistle, purslane, raspberry, blackcurrant, hazelnut, almond, wheat germ, borage, evening primrose (rose mallow) and poppy, stones, pips and seeds, of argan, palm, copra, sesame, olive, castor oil, coconut, Brazil nut. jatropha, babassu, karanja, neem, mahua, saijar, jojoba, tung, tall or tall oil, algae, used frying oils, fish fat oils, oils and fats of bovines, pigs, goats, equidae and poultry, as well as mixtures of two or more thereof.

9. Method according to any one of claims 5 to 8, **characterised in that** the fatty substance comprises from 0.1% to 100%, preferably from 1% to 99%, preferably again from 3% to 95%, entirely preferably from 5 to 90% by weight of at least one free fatty acid.

10. Method according to any one of claims 5 to 9, **characterised in that** the free fatty acids are chosen from the carboxylic acids having from 3 to 32 carbon atoms, saturated or unsaturated, linear or branched, optionally including one or more rings, and in particular the carboxylic acids chosen from propionic acid (3), butyric acid (4), caproic acid (6), caprylic acid (8), pelargonic acid (9), capric acid (10), undecanoic acid (11), lauric acid (12), myristic acid (14), myristoleic acid (14:1), palmitic acid (16), palmitoleic acid (16:1), margaric acid (17), stearic acid (18), oleic acid (18:1), ricinoleic acid (18:1), elaidic acid (18:1), petroselinic acid (18:1), vaccenic acid (18:1), linoleic acid (18:2), linolenic acids (18:3) and isomers thereof, licanic acid (18:3), arachidic acid (20), gadoleic acid (20:1), arachidonic acid (20:4), behenic acid (22), erucic acid (C22:1), as well as mixtures of two or more thereof, in any proportions.

11. Method according to any one of claims 5 to 10, **characterised in that** the alcohol is chosen from monoalcohols, diols, alkyl triols, linear, branched or cyclic, preferably linear or branched, preferably again linear, comprising from 1 to 6 carbon atoms, preferably from 1 to 4 carbon atoms, and the mixtures of two or more thereof, in any proportions.

12. Method according to claim 11, **characterised in that** the alcohol is chosen from methanol, ethanol, propanol, ethylene glycol, and glycerol and mixtures thereof, and preferably the alcohol is methanol or glycerol.

13. Method for preparing biodiesel, **characterised in that** it uses the composition as defined in claim 3 or 4.

14. Method according to claim 13, **characterised in that** it includes the steps of the method as defined in any one of claims 5 to 12.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- FR 2929621
- US 3071604 A
- US 3887488 A
- WO 9501331 A1
- WO 2006043281 A
- US 20030167681 A